# EUROPÄISCHE PATENTSCHRIFT

(11) **EP 0 824 909 B1**
(45) Veröffentlichungstag und Bekanntmachung des Hinweises auf die Patenterteilung: **10.09.2003**
(21) Anmeldenummer: 97810572.4
(22) Anmeldetag: 15.08.1997
(51) Int. Cl.: A61K 7/06, A61K 7/42

(54) **Verwendung von ausgewählten Benzotriazol- und Triazinderivaten zum Schützen von menschlichem Haar vor der schädigenden Einwirkung von UV-Strahlung**
Use of specified benzotriazole and triazine derivatives for protecting human hair against damage by U.V. rays
Utilisation des dérivés de benzotriazole et de triazine pour la protection contre les rayons U.V. des cheveux humains

(30) Priorität: 23.08.1996 CH 207096; 27.06.1997 CH 156197
(43) Veröffentlichungstag der Anmeldung: 25.02.1998
(73) Patentinhaber: Ciba Specialty Chemicals Holding Inc., 4057 Basel (CH)
(72) Erfinder: Luther, Helmut, 79639 Grenzach-Wyhlen (DE); Baudin, René, 4057 Basel (CH); Stehlin, Albert, 68300 Rosenau (FR); Haase, Jürg, 4126 Bettingen (CH)

(56) Entgegenhaltungen:
- EP-A- 0 743 309
- GB-A- 2 286 774
- US-A- 4 045 549
- PATENT ABSTRACTS OF JAPAN vol. 011, no. 018 (C-398), 17. Januar 1987 (1987-01-17) & JP 61 192781 A (TOYOTA CENTRAL RES & DEV LAB INC), 27. August 1986 (1986-08-27)

## Beschreibung

Die vorliegende Erfindung betrifft die Verwendung von ausgewählten Benzotriazol- und Triazinderivaten zum Schützen von menschlichem Haar vor der schädigenden Einwirkung von UV-Strahlung.

Wird Human-Haar über längere Zeit dem Sonnenlicht ausgesetzt, können verschiedene Schädigungen auftreten. Mit Farbstoffen gefärbtes Haar kann sich in Farbe und Farbton unter dem Einfluss von Sonnenlicht verändern. Blondes Haar wird gelblich. Die Oberfläche des Haars wird rauher und gleichzeitig trockener. Ausserdem verliert es mit der Zeit seinen Glanz.

Durch die Verwendung von UV-Absorbern können natürliche und gefärbte Haare wirkungsvoll vor schädlichen Sonnenstrahlen geschützt werden. Leider besitzen die bisher verwendeten UV-Absorber ein auf Human-Haar unzureichendes Aufziehvermögen, d.h. sie lassen sich leicht herauswaschen und haben daher nur eine kurzzeitige Wirkung.

Überraschenderweise wurde nun gefunden, dass gewisse Benzotriazol- und Triazinderivate eine sehr gute Substantivität gegenüber Human-Haar aufweisen und gleichzeitig einen wirkungsvollen UV-Schutz für das Haar bilden.

Gegenstand der vorliegenden Erfindung ist daher die Verwendung von ausgewählten Benzotriazol- und Triazinderivaten zum Schützen von menschlichem Haar vor der schädigenden Einwirkung von UV-Strahlung.

Bei den ausgewählten Benzotriazolverbindungen handelt es sich um 3-[2'H-Benzotriazol-(2')-yl]-4-hydroxybenzolsulfonsäuren, die der Formel 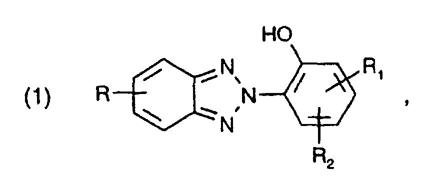 entsprechen.

Bei den ausgewählten Triazinderivaten handelt es sich um Verbindungen der Formel

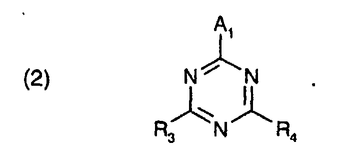

In den Formeln (1) und (2) bedeuten
- R: Wasserstoff oder Chlor,
- R₁: C₁-C₁₂-Alkyl oder Phenyl-C₁-C₃-Alkyl;
- R₂: C₁-C₄-Alkyl; oder SO₃M;
- A₁: einen Rest der Formel 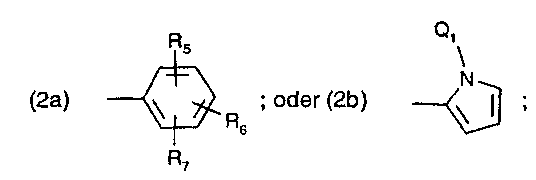
- R₃: einen Rest der Formel 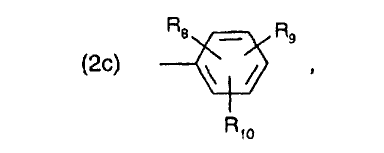
- R₄: Halogen; oder einen Rest der Formel 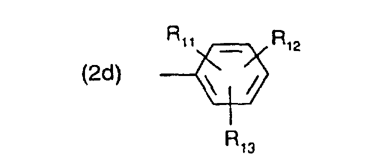
- Q₁: C₁-C₁₈-Alkyl;
- R₅, R₆ und R₇,: unabhängig voneinander, Wasserstoff; C₁-C₁₂-Alkyl; C₁-C₁₈-Alkoxy; Halogen; Hydroxy; oder einen Rest der Formel 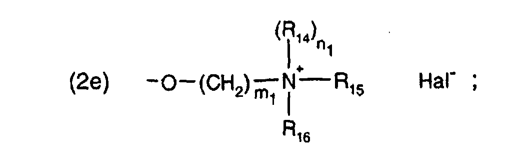
- R₈, R₉, R₁₀, R₁₁, R₁₂ und R₁₃,: unabhängig voneinander, Wasserstoff, Hydroxy; C₁-C₈-Alkyl; C₁-C₈-Alkoxy; Phenyl-C₁-C₃-Alkyl; C₅-C₇-Cyclohexyl; oder -SO₃M;
- R₁₄, R₁₅ und R₁₆,: unabhängig voneinander, C₁-C₅-Alkyl; oder, wenn n₁ = 0 bedeutet, R₁₅ und R₁₆ zusammen mit dem an die Reste gebundenen Stickstoffatom einen heteroaromatischen sechsgliedrigen Rest;
- M: Wasserstoff; Natrium; oder Kalium;
- Hal: Halogenatom;
- m₁: 1 bis 5; und
- n₁: 0 oder 1.

Bei den C₁-C₁₂-Alkylresten kann es sich um die Gruppen Methyl, Ethyl, Propyl, iso-Propyl, Butyl, iso-Butyl, sec. Butyl, tert. Butyl, Pentyl, Hexyl, Octyl, Decyl oder Dodecyl handeln.

C₁-C₁₈-Alkoxy sind geradkettige oder verzweigte Reste wie z.B. Methoxy, Ethoxy, Propoxy, Butoxy, Pentyloxy, Hexyloxy, Heptyloxy, Octyloxy, Isooctyloxy, Nonyloxy, Undecyloxy, Dodecyloxy, Tetradecyloxy oder Pentadecyloxy, Hexadecyloxy, Heptadecyloxy oder Octadecyloxy.

Bei dem Phenyl-C₁-C₃-Alkylrest handelt es sich z.B. um den Benzyl-, Phenethyl- oder 2,2-Dimethylbenzylrest.

Halogen bedeutet Fluor, Brom oder vorzugsweise Chlor.

Als Benzotriazolderivate kommen vorzugsweise Verbindungen der Formel (1) in Betracht, worin
- R: Wasserstoff und
- R₁: C₁-C₁₂-Alkyl;
bedeuten.

Besonders bevorzugt sind Benzotriazole der Formel 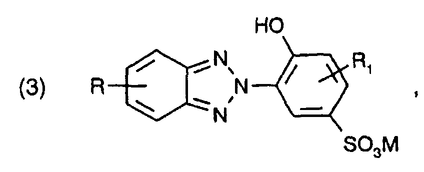 worin
R, R₁ und M die oben angegebene Bedeutung haben.

Ganz besonders bevorzugt sind Verbindungen der Formel 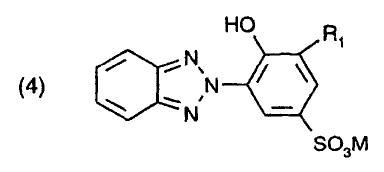 worin
- R₁: C₁-C₁₂-Alkyl; und
- M: Wasserstoff; Natrium; oder Kalium;
bedeuten.

Bedeutet R₅, R₆ oder R₇ einen Rest der Formel (2e), so entspricht dieser Rest vorzugsweise der Formel 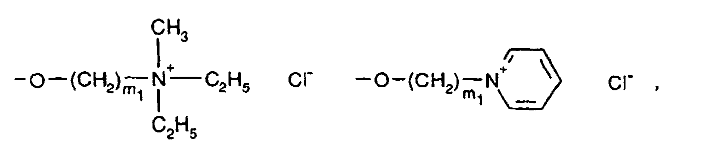 worin
- m₁: die angegebene Bedeutung hat.

Bevorzugte Hydroxyphenyl-s-triazine entsprechen der Formel 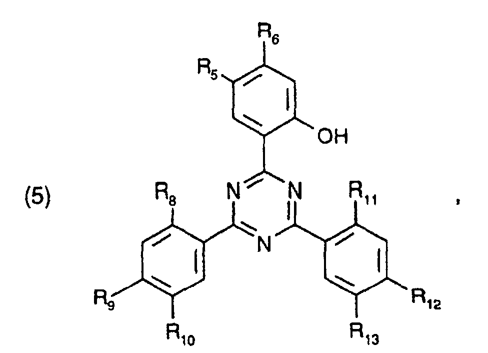 worin
- R₅: Wasserstoff; oder SO₃M;
- R₆: Wasserstoff; Hydroxy; C₁-C₅-Alkoxy; oder einen Rest der Formel (2e);
- R₈, R_{9,} R₁₁ und R₁₂,: unabhängig voneinander, Wasserstoff oder Hydroxy; und
- R₁₀ und R₁₃,: unabhängig voneinander, Wasserstoff; oder Sulfo;
bedeuten.

Von besonderem Interesse sind Verbindungen der Formel (2), die mindestens eine Sulfogruppe oder einen Rest der Formel (2e) aufweisen.

Von ganz besonderem Interesse sind Hydroxyphenyl-s-triazine der Formel (5), worin
- R₅, R₈, R₉, R₁₀, R₁₁, R₁₂ und R₁₃: Wasserstoff; und
- R₆: einen Rest der Formel (2e);
bedeuten.

Weiterhin sind Triazinverbindungen der Formel (5) bevorzugt, worin
- R₅: Wasserstoff; oder SO₃M;
- R₆: Hydroxy oder C₁-C₅-Alkoxy;
- R₈, R₉, R₁₁ und R₁₂: Hydroxy;
- R₁₀ und R₁₃: SO₃M; und
- M: Wasserstoff; Natrium; oder Kalium;
bedeuten.

Beispielhafte Verbindungen für erfindungsgemäss einsetzbare Triazinderivate sind: 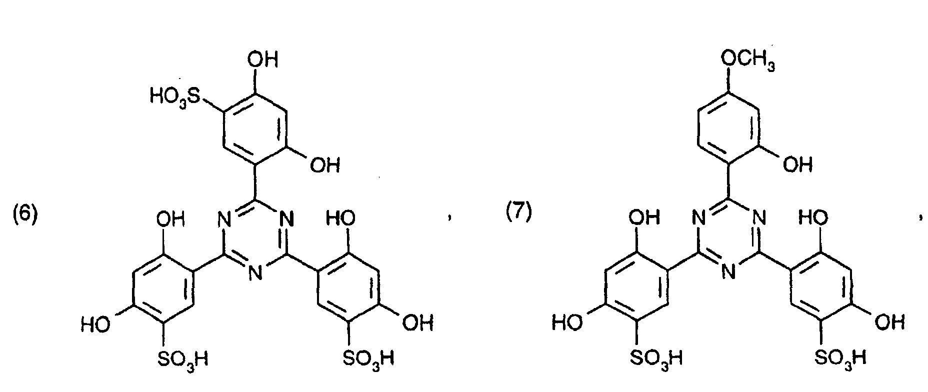 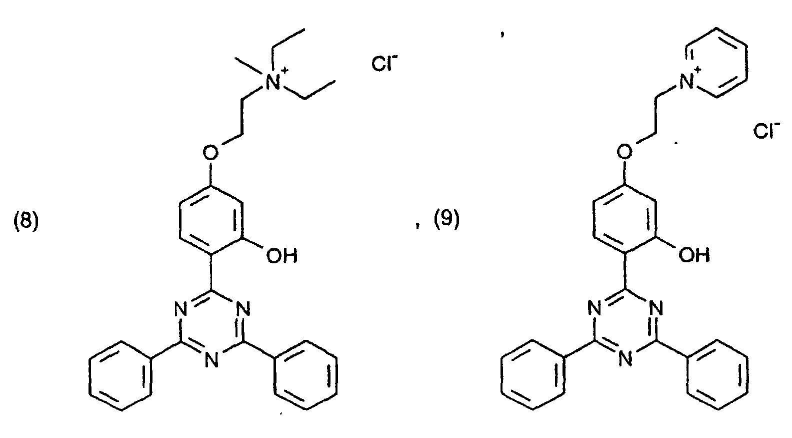 oder

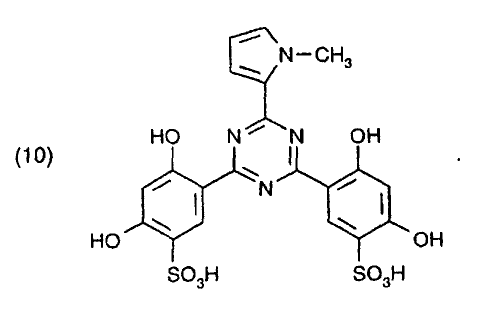

Erfindungsgemäss können auch Gemische von UV-Absorbern der Formeln (1) und (2) bzw. (3) verwendet werden.

Die Benzotriazolderivate der Formeln (1), (3) und (4) stellen bekannte Verbindungen dar. Natriumsalze von 3-[2'H-Benzotriazol-(2')-yl]-4-hydroxybenzolsulfonsäuren sind z.B. aus der EP-A-0,112,120 bekannt. Sie werden durch Sulfonierung der entsprechenden 2-[2'H-Benzotriazol-(2')-yl]-phenole mit Chlorsulfonsäure hergestellt, wobei diese Ausgangsstoffe gemäss dem Verfahren von J. Rosevear und J.F.K: Wilshire, Aust. J. Chem. 1985, 38, 1163-1176 hergestellt werden.

Die Triazinderivate der Formeln (2) und (5) bis (9) sind ebenfalls literaturbekannt und können in an sich bekannter Weise hergestellt werden, so z.B. durch Erhitzen eines Amidins und eines o-Hydroxybenzolcarbonsäureesters, vorzugsweise im etwa molaren Mengenverhältnis von 2:1 in siedenden, organischen Lösungsmitteln [cf. US-A-3,896,125 und Helv.Chim. Acta 55 (1972), 1566-1595].

Die erfindungsgemäss verwendeten 3-[2'H-Benzotriazol-(2')-yl]-4-hydroxybezolsulfonsäuren der Formel (1), (3) und (4) und die 2-(2'-Hydroxyphenyl)-s-triazine der Formeln (2) und (5) sind als UV-Absorber für den technischen Einsatz, z.B. für Kunststoffe, Lacke und Filme, Natur- oder Kunstharze, wachshaltige Stoffe, Kautschuk oder auch als Lichtschutzmittel in hautkosmetischen Präparaten bekannt.

JP 61192781 beschreibt die Verwendung von Benzotriazolen als Absorber in Farben. Aus GB 2286774 ist die Verwendung von Triazinen in pharmazeutischen und kosmetischen Zubereitungen bekannt.

Die erfindungsgemäss eingesetzten Benzotriazol- und Triazinderivate zeichnen sich dadurch aus, dass sie
- eine hohe Substantivität zu Human-Haar und haben und
- einen hohen UV-Schutz für das Haar garantieren.

Einen weiteren Erfindungsgegenstand bildet ein haarkosmetische Formulierung, enthaltend mindestens 0,25 bis 5 Gew.%, bezogen auf das Gesamtgewicht der Zusammensetzung, eines UV-Absorbers der Formeln (1) und/oder (2) sowie mindestens einen haut- und haarverträglichen Hilfsstoff.

Die haarkosmetische Formulierung kann durch physikalisches Mischen des oder der UV-Absorber mit dem Hilfsstoff oder den Hilfsstoffen durch gewöhnliche Methoden, wie z.B. durch einfaches Zusammenrühren der Einzelkomponenten erfolgen.

Für die haarkosmetische Verwendung haben die erfindungsgemässen UV-Absorber, sofern sie nicht wasserlöslich sind, gewöhnlich eine mittlere Partikelgrösse im Bereich von 0,02 bis 2, vorzugsweise 0,05 bis 1,5, und ganz besonders von 0,1 bis 1,0 µ. Die unlöslichen erfindungsgemässen UV-Absorber können durch übliche Methoden, z.B. Mahlen mit z.B. einer Düsen-, Kugel-, Vibrations- oder Hammermühle auf die gewünschte Partikelgrösse gebracht werden. Vorzugsweise wird das Mahlen in Anwesenheit von 0,1 bis 30, vorzugsweise 0,5 bis 15 Gew.-%, bezogen auf den UV-Absorber, einer Mahlhilfe, wie z.B. eines alkylierten Vinylpyrrolidon-Polymers, eines Vinylpyrrolidon-Vinylacetat-Copolymers, eines Acylglutamates oder insbesondere eines Phospholipids durchgeführt.

Für die Herstellung der erfindungsgemässen haarkosmetischen Formulierungen kann jeder konventionell einsetzbare Emulgator verwendet werden, wie z.B. einer oder mehrere ethoxylierte Ester von natürlichen Derivaten, wie z.B. polyethoxylierte Ester von hydrogeniertem Castor-ÖI; oder ein Silikonöl-Emulgator wie z.B. Silikonpolyol; eine gegebenenfalls ethoxylierte Fettsäureseife; ein ethoxylierter Fettalkohol; ein gegebenenfalls ethoxylierter Sorbitanester; eine ethoxylierte Fettsäure; oder ein ethoxyliertes Glycerid.

Die haarkosmetische Formulierung kann dabei
- in Form eines Shampoo, einer Lotion, eines Gels oder einer Emulsion zur Spülung, vor oder nach dem Schampunieren, vor oder nach einer Färbung oder Entfärbung, vor oder nach dem Legen einer Dauerwelle oder einem Entfrisiervorgang,
- in Form einer Lotion, eines Schaums oder eines Gels zum Frisieren oder Behandeln,
- in Form einer Lotion oder eines Gels zum Bürsten oder zur Wellengebung,
- in Form eines Haarlacks,
- in Form einer Zusammensetzung zur Dauerwelle oder zum Entfrisieren, zur Färbung oder Entfärbung der Haare vorliegen.

Es können zum Beispiel die folgenden haarkosmetischen Formulierungen verwendet werden:
a₁) spontan emulgierende Stammformulierung, bestehend aus dem UV-Absorber, PEG-6-C₁₀-Oxoalkohol und Sorbitanesquioleat, das mit Wasser und einer beliebigen quaternären Ammoniumverbindung, wie z.B. 4% Minkamidopropyl-dimethyl-2-hydroxyethylammoniumchlorid oder Quaternium 80 versetzt wird;
a₂) spontan emulgierende Stammformulierung, bestehend aus dem UV-Absorber, Tributylcitrat und PEG-20-Sorbitanmonooleat, das mit Wasser und einer beliebigen quaternären Ammoniumverbindung, wie z.B. 4% Minkamidopropyl-dimethyl-2-hydroxyethylammoniumchlorid oder Quaternium 80 versetzt wird;
b) Quatdotierte Lösungen des UV-Absorbers in Butyltriglykol und Tributylcitrat;
c) Dispersionen von auf nach bekannten Methoden (Fällen aus Lösungen oder Lösungsgemischen, Mahlungen) erhaltenen mikronisierten UV-Absorbern mit einem mittleren Durchmesser von 0,05 - 1,0 µm in APG ( z.B. Plantaren) und einem Quat (z.B. Minkamidopropyl-dimethyl-2-hydroxyethylammoniumchlorid) in einer wässrigen Zubereitung;
d) Mischungen oder Lösungen des UV-Absorbers mit n-Alkylpyrrolidon.

Vorzugsweise werden haarkosmetischen Formulierungen verwendet, die als UV-Absorber eine Benzotriazolverbindung der Formel (1), Triethylenglykolbutylether und Tributylcitrat enthalten.

Unter diesen Formulierungen sind ganz besonders diejenigen bevorzugt, die als UV-Absorber eine Benzotriazolverbindung der Formel (4) enthalten.

Die letztgenannten Formulierungen enthalten insbesondere

| | |
|---|---|
| 25 bis 50 Teile | der Benzotriazolverbindung der Formel (1), insbesondere der Formel (4), |
| 40 bis 80 Teile | Triethylenglykolbutylether und |
| 0,5 bis 5 Teile | Tributylcitrat. |

Die haarkosmetische Formulierung kann neben den erfindungsgemässen UV-Absorbern auch noch einen oder mehrere weitere UV-Schutzstoffe der folgenden Substanzklassen enthalten:
1. p-Aminobenzoesäurederivate, wie z.B. 4-Dimethylaminobenzoesäure-2-ethylhexylester;
2. Salicylsäurederivate, wie z.B. Salicylsäure-2-ethylhexylester;
3. Benzophenonderivate, wie z.B. 2-Hydroxy-4-methoxybenzophenon und sein 5-sulfonsäurederivat;
4. Dibenzoylmethanderivate, wie z.B. 1-(4-tert.-Butylphenyl)-3-(4-methoxyphenyl)propan-1,3-dion;
5. Diphenylacrylate, wie z.B. 2-Ethylhexyl-2-cyano-3,3-diphenyl acrylat und 3-(Benzofuranyl)-2-cyanoacrylat;
6. 3-lmidazol-4-yl-acrylsäure und -ester;
7. Benzofuranderivate, insbesondere 2-(p-Aminophenyl)benzofuranderivate, beschrieben in der EP-A-582,189, US-A-5,338,539, US-A-5,518,713 und der EP-A-613,893;
8. polymere UV-Absorber wie z.B. die in der EPA-709,080 beschriebenen Benzylidenmalonatderivate;
9. Zimtsäurederivate, wie z.B. die in der US-A-5,601,811 und WO 97/00851 offenbarten 4-Methoxyzimtsäure-2-ethylhexylester bzw. lsoamylester oder Zimtsäurederivate;
10. Campherderivate, wie z.B. 3-(4'-Methyl)benzyliden-bornan-2-on, 3-Benzyliden-bornan-2-on, N-[2(und 4)-2-Oxyborn-3-yliden-methyl)-benzyl]acrylamid-Polymer, 3-(4'-Trimethylammonium)-benzyliden-bornan-2-on methylsulfat, 3,3'-(1,4-Phenylendimethin)-bis(7,7-dimethyl-2-oxo-bicyclo-[2.2.1]heptan-1-methansulphonsäure) und Salze, 3-(4'-Sulfo)-benzyliden-bornan-2-on und Salze;
11. Trianilino-s-Triazinderivate, wie z.B. 2,4,6-Trianilin-(p-carbo-2'-ethyl-1'-oxi)-1,3,5-triazin sowie die in der US-A-5,332,568, EP-A-517,104, EP-A-507,691, WO 93/17002 und EP-A-570,838 offenbarten UV-Absorber;
12. 2-Hydroxyphenyl-Benzotriazol-Derivate;
13.2-Phenylbenzimidazol-5-sulfonsäure und deren Salze;
14. Menthyl-o-aminobenzoat.
15. TiO₂ (unterschiedlich umhüllt), ZnO und Mica.

Auch die in "Sunscreens", Eds. N.J. Lowe, N.A.Shaath, Marcel Dekker, Inc., New York and Basel oder in Cosmetics & Toiletries (107), 50ff (1992) beschriebenen UV-Absorber können als zusätzliche UV-Schutzstoffe in der erfindungsgemässen Haar-Formulierung verwendet werden.

Weiterhin können die haarkosmetischen Formulierungen auch zusammen mit bekannten Antioxidantien, wie z.B. Vitamin E, Carotinoiden oder HALS-Verbindungen eingesetzt werden.

Weiterhin können die erfindungsgemässen Zusammensetzungen noch weitere nützliche Hilfsmittel, wie z.B. oberflächenaktive Mittel, Verdickungsmittel, Polymere, Konservierungsmittel, Duftstoffe, Schaumstabilisatoren, Elektrolyte, organische Lösungsmittel, Öle, Wachse, Entfettungsmittel, Farbstoffe und/oder Pigmente enthalten, die dazu dienen, der haarkosmetischen Zusammensetzung die gleiche Tönung wie das zu behandelnde Haar zu verleihen sowie weitere, in der Haarkosmetik übliche Hilfsstoffe.

Die vorliegende Erfindung betrifft auch ein Verfahren zur Behandlung von Human-Haar zum Schützen vor der schädigenden Einwirkung von UV-Strahlung. Das Verfahren ist dadurch gekennzeichnet, dass man das Haar mit einem Shampoo, einer Lotion, einem Gel oder einer Emulsion zur Spülung, vor oder nach dem Schampunieren, vor oder nach einer Färbung oder Entfärbung, vor oder nach dem Legen einer Dauerwelle oder einem Entfrisiervorgang, mit einer Lotion, einem Schaum oder einem Gel zum Frisieren, mit einer Lotion, einem Schaum oder einem Gel zum Bürsten oder zu Wellengebung, mit einem Haarlack, mit einer Zusammensetzung zur Dauerwelle oder zum Entfrisieren, zur Färbung oder Entfärbung der Haare behandelt, wobei das Shampoo, die Lotion, das Gel, die Emulsion, der Schaum, der Haarlack oder die Zusammensetzung zur Dauerwelle, zum Entfrisieren, zur Färbung oder Entfärbung der Haare mindestens einen UV-Absorber der Formel (1) oder (2) enthält.

Die folgenden Beispiele dienen der Veranschaulichung der Erfindung, ohne sie darauf zu beschränken.

### Beispiel 1: Bestimmung der Affinität von UV-Absorbern auf Haaren durch Bestimmung des Ausziehgrades aus Formulierungen oder Lösungen

10 ml einer 10 mmolaren UV-Absorberlösung und 1g Haarsträhne werden in einem Glasgefäss definiert und reproduzierbar geschüttelt. Mittels Spektralphotometer wird die Konzentrationsabnahme des UV-Absorbers in der Testformulierung bzw. -Lösung nach 30 und 60 Minuten bestimmt (siehe Tabelle 1). Der so bestimmte Ausziehgrad stellt ein Mass für die Substantivität des UV-Absorbers bzw. der Formulierung, in der er enthalten ist, dar.

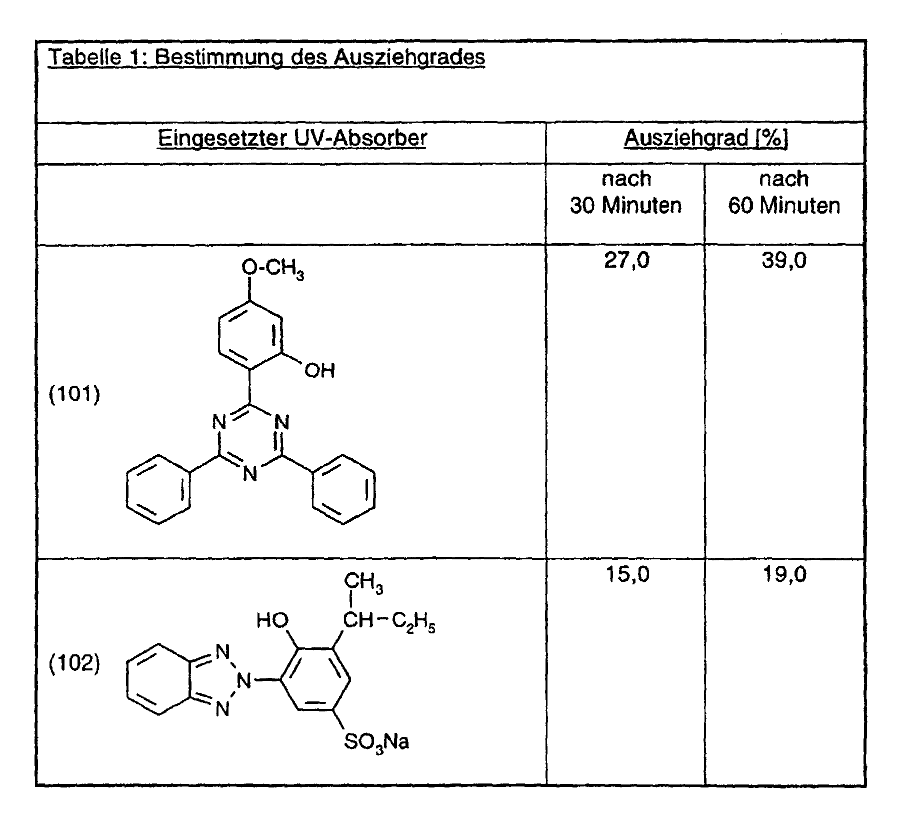

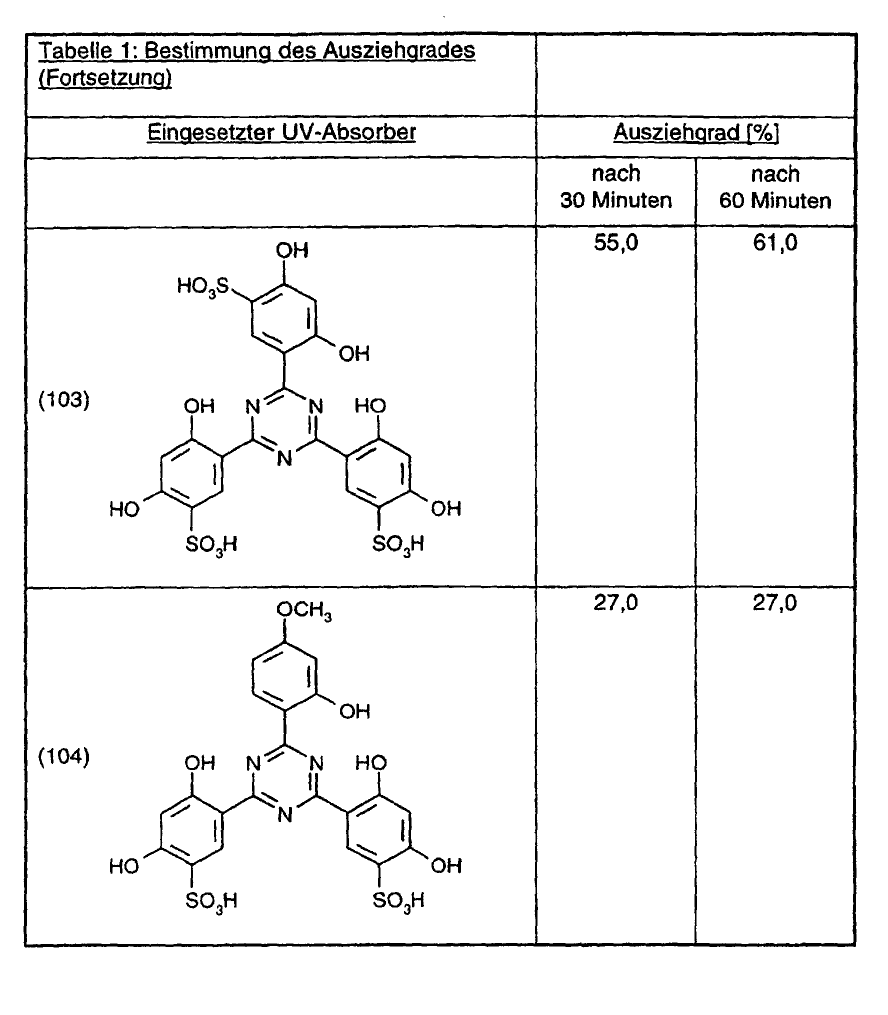

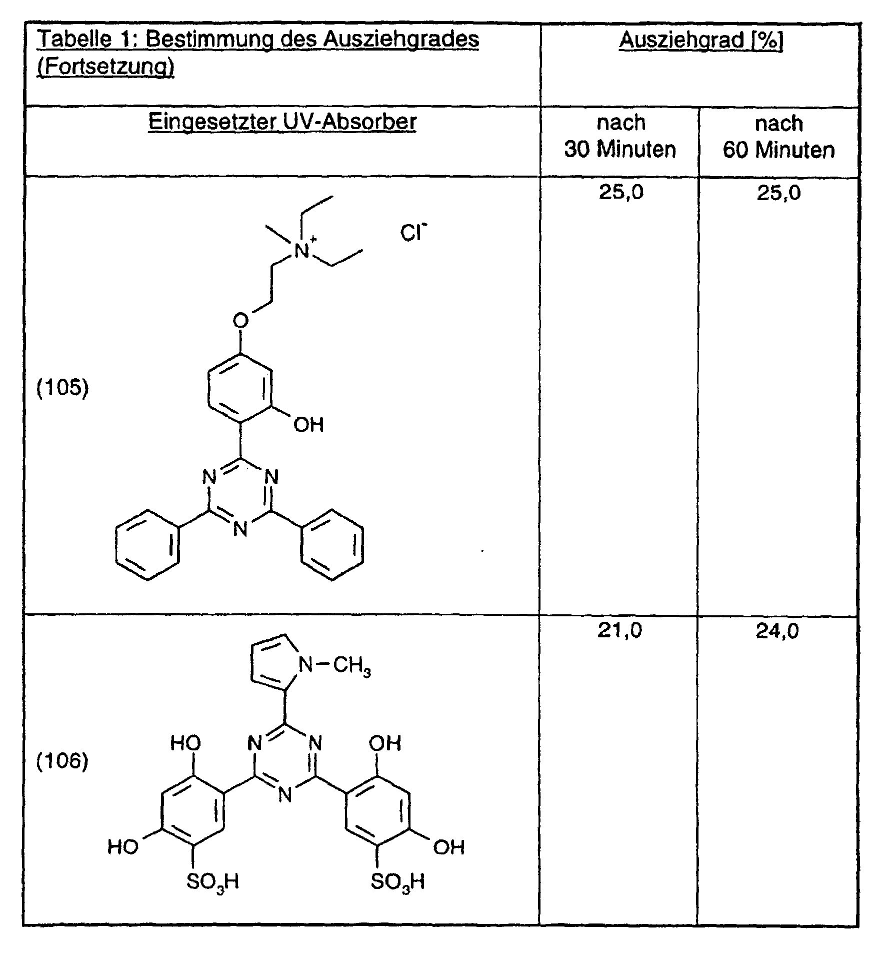

### Beispiel 2: Herstellung einer Haarspülung:

| Zusammensetzung | [g] |
|---|---|
| UV-Absorber der Formel (102) | 1 |
| Mischung aus Cetylstearylalkohol und dem Anlagerungsprodukt aus 1 Mol Cetylstearylalkohol und 33 Mol Ethylenoxid | 2 |
| Monoethanolamid | 0,5 |
| Xanthangum | 0,8 |
| Wasser | 95,7 |

Die einzelnen Komponenten werden gemischt, mit Konservierungsmittel und Parfümöl versetzt und anschliessend mit verdünnter HCI auf den pH-Wert 6,5 eingestellt.

Man erhält so eine gebrauchsfertige Haarspülung, die einen guten und anhaltenden UV-Schutz für das menschliche Haar bietet.

## Patentansprüche

1. Verwendung von ausgewählten Benzotriazol- und Triazinderivaten zum Schützen von menschlichem Haar vor der schädigenden Einwirkung von UV-Strahlung, **dadurch gekennzeichnet, dass** die ausgewählten Verbindungen der Formel 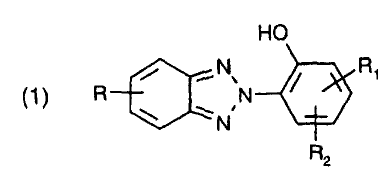 oder der Formel 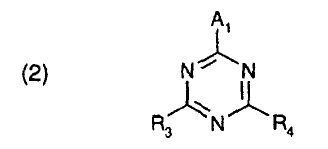 entsprechen, worin
R Wasserstoff oder Chlor,
R₁ C₁-C₁₂-Alkyl oder Phenyl-C₁-C₃-Alkyl;
R₂ C₁ bis C₄-Alkyl; oder SO₃M;
A₁ einen Rest der Formel 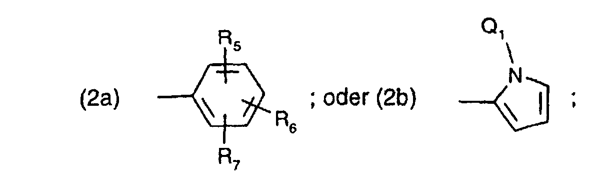
R₃ einen Rest der Formel 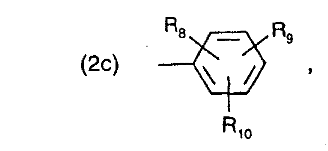
R₄ Halogen; oder einen Rest der Formel 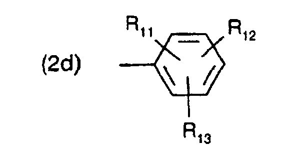
Q₁ C₁-C₁₈-Alkyl;
R₅, R₆ und R₇, unabhängig voneinander, Wasserstoff; C₁-C₁₂-Alkyl; C₁-C₁₈-Alkoxy; Halogen; Hydroxy; oder einen Rest der Formel 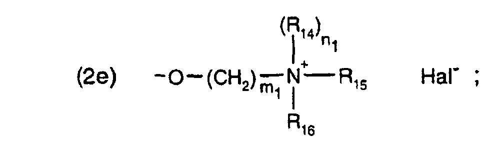
R₈, R₉, R₁₀, R₁₁, R₁₂ und R₁₃, unabhängig voneinander, Wasserstoff; Hydroxy; C₁-C₈-Alkyl; C₁-C₈-Alkoxy; Phenyl-C₁-C₃-Alkyl; C₅-C₇-Cyclohexyl; oder -SO₃M;
R₁₄, R₁₅ und R₁₆, unabhängig voneinander, C₁-C₅-Alkyl; oder, wenn n₁ = 0 bedeutet, R₁₅ und R₁₆ zusammen mit dem an die Reste gebundenen Stickstoffatom einen heteroaromatischen sechsgliedrigen Rest;
M Wasserstoff; Natrium; oder Kalium;
Hal Halogenatom;
m₁ 1 bis 5; und
n₁ 0 oder 1.

2. Verwendung nach Anspruch 1, **dadurch gekennzeichnet, dass** in den Verbindungen der Formel (1)
R Wasserstoff; und
R₁ C₁-C₁₂-Alkyl;
bedeuten.

3. Verwendung nach Anspruch 1 oder 2, **dadurch gekennzeichnet, dass** die ausgewählten Verbindungen der Formel 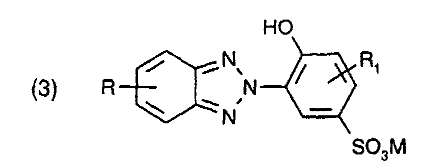 entsprechen, worin
R, R₁ und M die in Anspruch 1 angegebene Bedeutung haben.

4. Verwendung nach einem der Ansprüche 1 bis 3, **dadurch gekennzeichnet, dass** Verbindungen der Formel 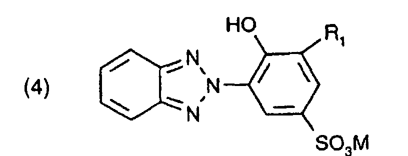 verwendet werden, worin
R₁ C₁-C₁₂-Alkyl; und
M Wasserstoff; Natrium; oder Kalium;
bedeuten.

5. Verwendung nach Anspruch 1, **dadurch gekennzeichnet, dass** Formel (2e) den Rest der Formel 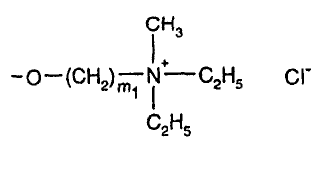 oder den Rest der Formel 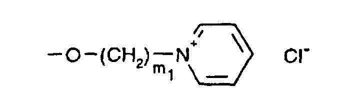 bedeutet, worin
m₁ die in Anspruch 1 angegebene Bedeutung hat.

6. Verwendung nach 1, **dadurch gekennzeichnet, dass** Verbindungen der Formel (2) verwendet werden, die mindestens eine Sulfogruppe oder einen Rest der Formel (2e) aufweisen.

7. Verwendung nach Anspruch 1, 5 oder 6, **dadurch gekennzeichnet, dass** Hydroxyphenyl-s-triazine der Formel 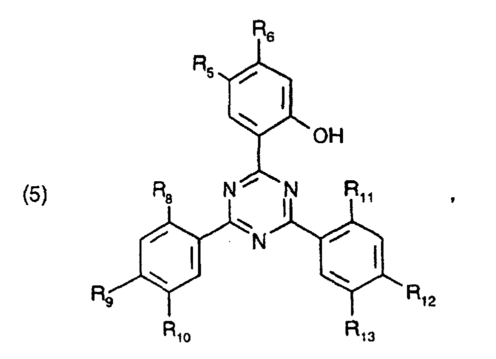 verwendet werden, worin
R₅ H; oder SO₃M;
R₆ Wasserstoff; Hydroxy; C₁-C₅-Alkoxy; oder einen Rest der Formel (2e);
R₈, R_{9,} R₁₁ und R₁₂, unabhängig voneinander, Wasserstoff oder Hydroxy; und
R₁₀ und R₁₃, unabhängig voneinander, Wasserstoff; oder Sulfo;
bedeuten.

8. Verwendung nach Anspruch 7, **dadurch gekennzeichnet, dass** Verbindungen der Formel (5) verwendet werden, worin
R₅, R₈, R₉, R₁₀, R₁₁, R₁₂ und R₁₃ Wasserstoff; und
R₆ einen Rest der Formel (2e);
bedeuten.

9. Verwendung nach Anspruch 7, **dadurch gekennzeichnet, dass** Verbindungen der Formel (5) verwendet werden, worin
R₅ Wasserstoff; oder SO₃M;
R₆ Hydroxy oder C₁-C₅-Alkoxy;
R₈, R₉, R₁₁ und R₁₂ Hydroxy;
R₁₀ und R₁₃ SO₃M; und
M Wasserstoff; Natrium; oder Kalium;
bedeuten.

10. Verwendung nach einem der Ansprüche 1 bis 9, **dadurch gekennzeichnet, dass** Gemische von UV-Absorbern der Formel (1) und (2) verwendet werden.

11. Haarkosmetische Formulierung, enthaltend mindestens 0,25 bis 5 Gew.%, bezogen auf das Gesamtgewicht der Zusammensetzung, eines UV-Absorbers der Formel (1) und/oder (2) sowie mindestens einen haut- und haarverträglichen Hilfsstoff.

12. Formulierung nach Anspruch 11, enthaltend als UV-Absorber eine Benzotriazolverbindung der Formel (1), Triethylenglykolbutylether und Tributylcitrat.

13. Formulierung nach Anspruch 12, **dadurch gekennzeichnet, dass** sie als UV-Absorber eine Benzotriazolverbindung der Formel (4) enthält.

14. Formulierung nach Anspruch 12 oder 13, **dadurch gekennzeichnet, dass** sie
| | |
|---|---|
| 25 bis 50 Teile | der Benzotriazolverbindung der Formel (1), insbesondere der Formel (4), |
| 40 bis 80 Teile | Triethylenglykolbutylether und |
| 0,5 bis 5 Teile | Tributylcitrat |
enthält.

15. Formulierung nach einem der Ansprüche 11 bis 14, **dadurch gekennzeichnet, dass** sie als weitere UV-Schutzstoffe p-Aminobenzoesäurederivate, Salicylsäurederivate, Benzophenonderivate, Dibenzoylmethanderivate, Diphenylacrylate, Benzofuranderivate; Zimtsäurederivate, Camphorderivate, Trianilino-s-Triazinderivate, 2-Hydroxyphenyl-Benzotriazol-Derivate, 2-Phenylbenzimidazol-5-sulfonsäure und deren Salze, Menthyl-o-aminobenzoat, TiO₂ (unterschiedlich umhüllt), ZnO und Mica enthält.

16. Formulierung nach einem der Ansprüche 11 bis 15, **dadurch gekennzeichnet, dass** sie zusätzlich Antioxidantien enthält.

17. Verwendung der haarkosmetischen Formulierung gemäss einem der Ansprüche 11 bis 16 zum Schutz von natürlichen und gefärbten Human-Haaren vor ultravioletter Strahlung, **dadurch gekennzeichnet, dass** sie in Form eines Shampoos, einer Lotion, eines Gels oder einer Emulsion zur Spülung, vor oder nach dem Schampunieren, vor oder nach einer Färbung oder Entfärbung, vor oder nach dem Legen einer Dauerwelle oder einem Entfrisiervorgang, in Form einer Lotion, eines Schaums oder eines Gels zum Frisieren oder Behandeln, in Form einer Lotion, eines Schaums oder eines Gels zum Bürsten oder zur Wellengebung, in Form eines Haarlacks, in Form einer Zusammensetzung zum Legen einer Dauerwelle, zum Entfrisieren, zur Färbung oder Entfärbung der Haare vorliegt.

18. Verfahren zur Behandlung von natürlichen oder gefärbten Human-Haaren zum Schützen vor der schädigenden Einwirkung von UV-Strahlung, **dadurch gekennzeichnet, dass** man das Haar mit einem Shampoo, einer Lotion, einem Gel oder einer Emulsion zur Spülung, vor oder nach dem Schampunieren, vor oder nach einer Färbung oder Entfärbung, vor oder nach dem Legen einer Dauerwelle oder einem Entfrisiervorgang, mit einer Lotion, einem Schaum oder einem Gel zum Frisieren, mit einer Lotion, einem Schaum oder einem Gel zum Bürsten oder zur Wellengebung, mit einem Haarlack, mit einer Zusammensetzung zur Dauerwelle zum Entfrisieren, zur Färbung oder Entfärbung der Haare behandelt, **dadurch gekennzeichnet, dass** das Shampoo, die Lotion, das Gel, die Emulsion, der Schaum, der Haarlack oder die Zusammensetzung zur Dauerwelle, zum Entfrisieren, zur Färbung oder Entfärbung der Haare mindestens einen UV-Absorber der Formel (1) oder (2) enthält.

## Claims

1. Use of selected benzotriazole and triazine derivatives for protecting human hair from the harmful effects of UV radiation, wherein the selected compounds conform to formula 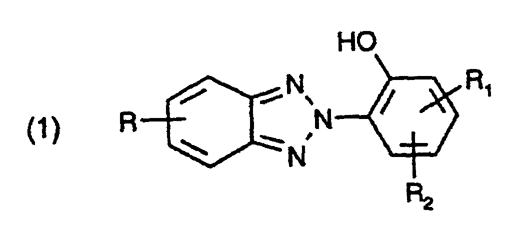 or to formula 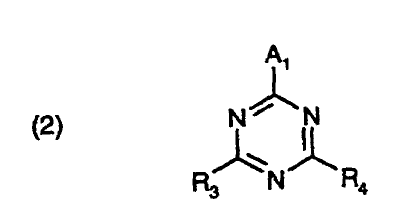 in which
R is hydrogen or chloro,
R₁ is C₁-C₁₂alkyl or phenyl-C₁-C₃alkyl;
R₂ is C₁- to C₄alkyl; or SO₃M;
A₁ is a radical of formula 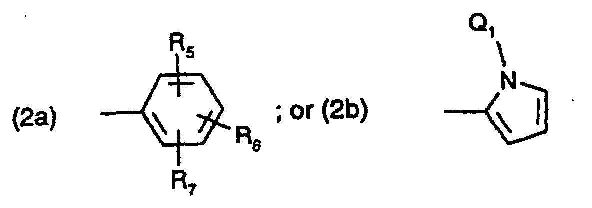
R₃ is a radical of formula 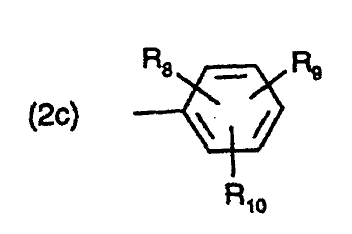
R₄ is halogen; or a radical of formula 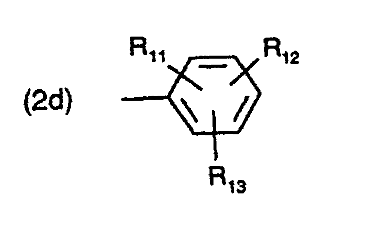
Q₁ is C₁-C₁₈alkyl;
R₅, R₆ and R₇ are each independently of one another hydrogen; C₁-C₁₂alkyl; C₁-C₁₈alkoxy; halogen; hydroxyl; or a radical of formula 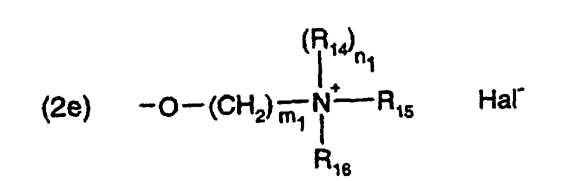 R₈, R₉, R₁₀, R₁₁, R₁₂ and R₁₃ are each independently of one another hydrogen; hydroxyl; C₁-C₈alkyl; C₁-C₈alkoxy; phenyl-C₁-C₃alkyl; C₅-C₇cyclohexyl; or -SO₃M;
R₁₄, R₁₅ and R₁₆ are each independently of one another C₁-C₅alkyl; or, if n₁ = 0, then R₁₅ and R₁₆, together with the nitrogen atom attached to the radicals, are a heteroaromatic six-membered radical;
M is hydrogen; sodium; or potassium;
Hal is a hydrogen atom;
m₁ is 1 to 5; and
n₁ is 0 or 1.

2. Use according to claim 1, wherein in the compounds of formula (1)
R is hydrogen; and
R1 is C₁-C₁₂alkyl.

3. Use according to claim 1 or 2, where the selected compounds conform to formula 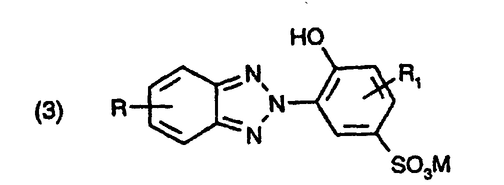 in which
R, R₁ and M have the meaning claimed in claim 1.

4. Use according to any one of claims 1 to 3, which comprises using compounds of formula 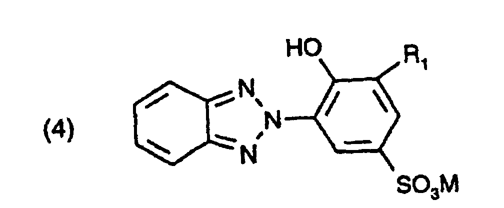 in which
R₁ is C₁-C₁₂alkyl; and
M is hydrogen; sodium; or potassium.

5. Use according to claim 1, wherein formula (2e) is the radical of formula 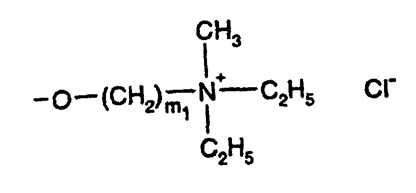 or the radical of formula 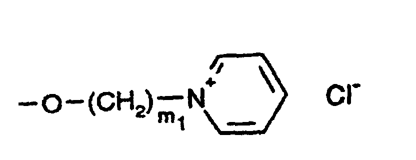 in which
m₁ has the meaning claimed in claim 1.

6. Use according to claim 1, which comprises using compounds of formula (2) which contain at least one sulfo group or a radical of formula (2e).

7. Use according to claim 1, 5 or 6, which comprises using hydroxyphenyl-s-triazines of formula 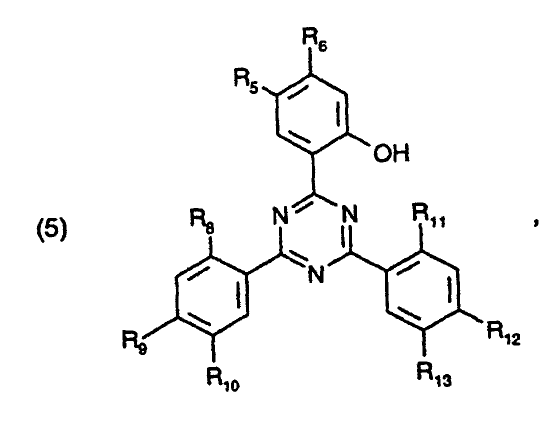 in which
R₅ is H; or SO₃M;
R₆ is hydrogen; hydroxyl; C₁-C₅alkoxy; or a radical of formula (2e);
R₈, R₉, R₁₁ and R₁₂ are each independently of one another hydrogen or hydroxyl; and R₁₀ and R₁₃ are each independently of the other hydrogen; or sulfo.

8. Use according to claim 7, which comprises using compounds of formula (5) in which R₅, R₈, R₉, R₁₀, R₁₁, R₁₂ and R₁₃ are hydrogen; and
R₆ is a radical of formula (2e).

9. Use according to claim 7, which comprises using compounds of formula (5) in which
R₅ is hydrogen; or SO₃M;
R₆ is hydroxyl or C₁-C₅alkoxy;
R₈, R₉, R₁₁ and R₁₂ are hydroxyl;
R₁₀ and R₁₃ are SO₃M; and
M is hydrogen; sodium; or potassium.

10. Use according to any one of claims 1 to 9, which comprises using mixtures of UV absorbers of formula (1) and (2).

11. A cosmetic formulation for hair, comprising at least 0.25 to 5% by weight, based on the total weight of the composition, of a UV absorber of formula (1) and/or (2) and at least one auxiliary compatible with skin and hair.

12. A formulation according to claim 11, comprising as UV absorber a benzotriazole compound of formula (1), triethylene glycol butyl ether and tributyl citrate.

13. A formulation according to claim 12, which comprises as UV absorber a benzotriazole compound of formula (4).

14. A formulation according to claim 12 or 13, which comprises
| | |
|---|---|
| 25 to 50 parts | of the benzotriazole compound of formula (1), especially of formula (4), |
| 40 to 80 parts | of triethylene glycol butyl ether, and |
| 0.5 to 5 parts | of tributyl citrate. |

15. A formulation according to any one of claims 11 to 14, which comprises as additional UV protectives p-aminobenzoic acid derivatives, salicylic acid derivatives, benzophenone derivatives, dibenzoylmethane derivatives, diphenylacrylates, benzofuran derivatives; cinnamic acid derivatives, camphor derivatives, trianilino-s-triazine derivatives, 2-hydroxyphenylbenzotriazole derivatives, 2-phenylbenzimidazole-5-sulfonic acid and the salts thereof, menthyl o-aminobenzoate, TiO₂ (variously coated), ZnO and mica.

16. A formulation according to any one of claims 11 to 15, which additionally comprises antioxidants.

17. Use of the cosmetic formulation for hair according to any one of claims 11 to 16 for protecting natural and dyed human hair from ultraviolet radiation, wherein it is used in the form of a shampoo, lotion or gel, or emulsion for rinsing, before or after shampooing, before or afer dyeing or decolorising, before or after a perming or straightening process, in the form of a lotion, foam or gel for setting or treating, in the form of a lotion, foam or gel for brushing or waving, in the form of a hair lacquer, in the form of a composition for perming or straightening hair or for dyeing or decolorising hair.

18. A method of treating natural or dyed human hair for protecting it from the harmful effects of UV radiation, which comprises treating the hair with a shampoo, lotion or gel, or with an emulsion for rinsing, before or after shampooing, before or after dyeing or decolorising, before or after a perming or straightening process, with a lotion, foam or gel for setting, with a lotion, foam or gel for brushing or waving, with a hair lacquer, with a composition for perming or straightening hair or for dyeing or decolorising hair, wherein the shampoo, lotion, gel, emulsion, foam, hair lacquer or composition for perming, straightening, dyeing or decolorising hair comprises at least one UV absorber of formula (1) or (2).

## Revendications

1. Utilisation de dérivés choisis du benzotriazole et de la triazine pour la protection de cheveux humains contre l'effet nocif du rayonnement UV, **caractérisée en ce que** les composés choisis répondent à la formule 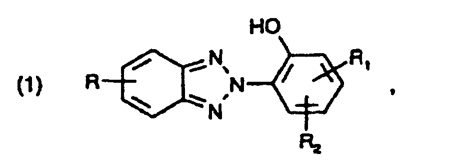 ou à la formule 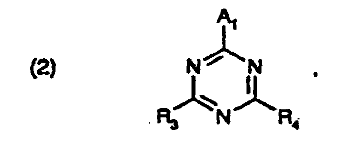
R représente un atome d'hydrogène ou de chlore ;
R₁ représente un groupe alkyle en C₁-C₁₂ ou phénylalkyle en C₁-C₃ ;
R₂ représente un groupe alkyle en C₁-C₄ ; ou SO₃M ;
A₁ représente un reste de formule 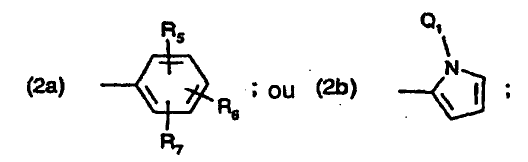
R₃ représente un reste de formule 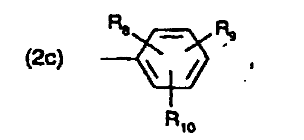
R₄ représente un atome d'halogène ; ou un reste de formule 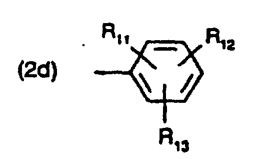
Q₁ représente un groupe alkyle en C₁-C₁₈ ;
R₅, R₆ et R₇ représentent, indépendamment les uns des autres, un atome d'hydrogène, un groupe alkyle en C₁-C₁₂ ; alkoxy en C₁-C₁₈ ; halogène ; hydroxy ; ou un reste de formule 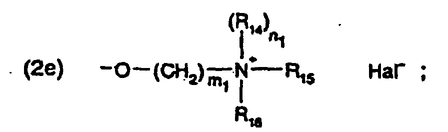
R₈, R₉, R₁₀, R₁₁, R₁₂ et R₁₃ représentent indépendamment les uns des autres, un atome d'hydrogène ; un groupe hydroxy ; un groupe alkyle en C₁-C₈ ; alkoxy en C₁-C₈ ; un groupe phénylalkyle en C₁-C₃ ; un groupe cyclohexyle en C₅-C₇ ; ou -SO₃M ;
R₁₄, R₁₅ et R₁₆ représentent, indépendamment les uns des autres, un groupe alkyle en C₁-C₅ ; ou lorsque n₁ = 0, R₁₅ et R₁₆ forment, conjointement avec l'atome d'azote lié aux restes, un reste hétéroaromatique à six chaînons ;
M représente un atome d'hydrogène ; de sodium ; ou de potassium ;
Hal représente un atome d'halogène ;
m va de 1 à 5 ; et
n₁ vaut 0 ou 1.

2. Utilisation selon la revendication 1, **caractérisée en ce que** dans les composés de formule (1)
R représente un atome d'hydrogène ; et
R₁ représente un groupe alkyle en C₁-C₁₂.

3. Utilisation selon la revendication 1 ou 2, **caractérisée en ce que** les composés choisis de formule 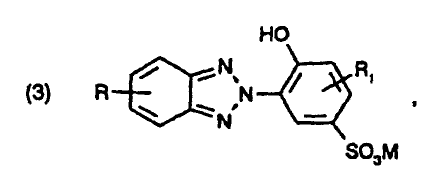 où R, R₁ et M possèdent la signification donnée à la revendication 1.

4. Utilisation selon l'une des revendications 1 à 3, **caractérisée en ce qu'**on utilise des composés de formule 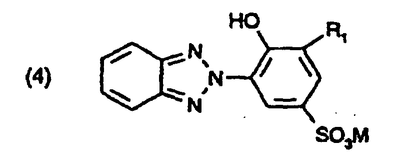 où
R₁ représente un groupe alkyle en C₁-C₁₂ ; et
M représente un atome d'hydrogène ; un atome de sodium ; ou de potassium.

5. Utilisation selon la revendication 1, **caractérisée en ce que** la formule (2e) correspond au reste de formule 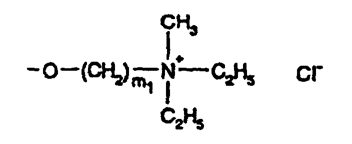 ou au reste de formule 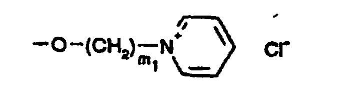 où
m₁ possède la signification donnée à la revendication 1.

6. Utilisation selon la revendication 1, **caractérisée en ce qu'**on utilise des composés de formule (2), qui présentent au moins un groupe sulfo ou un reste de formule (2e).

7. Utilisation selon la revendication 1, 5 ou 6, **caractérisée en ce qu'**on utilise des hydroxyphényl-s-triazines de formule 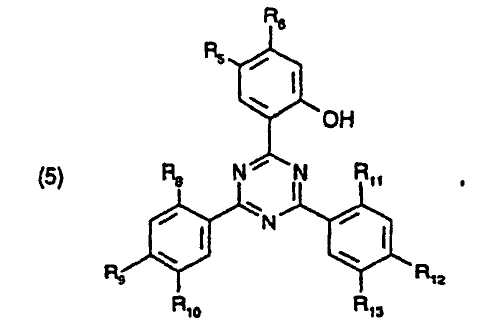 où
R₅ représente un atome d'hydrogène ; ou un groupe SO₃M ;
R₆ un atome d'hydrogène ; un groupe hydroxy ; groupe alkoxy en C₁-C₅ ; ou un reste de formule (2e) ;
R₈, R₉, R₁₁ et R₁₂ représentent, indépendamment les uns des autres, un atome d'hydrogène ou un groupe hydroxy ; et
R₁₀ et R₁₃ représentent, indépendamment l'un de l'autre, un atome d'hydrogène ; ou un groupe sulfo.

8. Utilisation selon la revendication 7, **caractérisée en ce qu'**on utilise des composés de formule (5), dans lesquels
R₅, R₈, R₉, R₁₀, R₁₁, R₁₂ et R₁₃ représentent un atome d'hydrogène ; et
R₆ représente un reste de formule (2e).

9. Utilisation selon la revendication 7, **caractérisée en ce qu'**on utilise des composés de formule (5), dans lesquels
R₅ représente un atome d'hydrogène ; ou un groupe SO₃M ;
R₆ représente un groupe hydroxy ou alkoxy en C₁-C_{5 ;}
R₈, R₉, R₁₁ et R₁₂ représentent un groupe hydroxy ; et
R₁₀ et R₁₃ représentent un groupe SO₃M ; et
M représente un atome d'hydrogène, un atome de sodium ; ou de potassium.

10. Utilisation selon l'une des revendications 1 à 9, **caractérisée en ce qu'**on utilise des mélanges d'absorbeurs d'UV de formule (1) et (2).

11. Formulation de cosmétique capillaire présentant une teneur de 0,25 à 5 % en masse, par rapport à la masse totale de la composition, en un absorbeur d'UV de formule (1) et/ou (2) ainsi qu'au moins un adjuvant toléré par la peau et le cheveu.

12. Formulation selon la revendication 11, renfermant en tant qu'absorbeur d'UV un composé du benzotriazole de formule (1), l'éther butylique de triéthylèneglycol et le citrate de tributyle.

13. Formulation selon la revendication 12, renfermant en tant qu'absorbeur d'UV un composé du benzotriazole de formule (4).

14. Formulation selon la revendication 12 ou 13, **caractérisée en ce qu'**elle renferme
| | |
|---|---|
| de 25 à 50 parties | du composé de benzotriazole de formule (1), en particulier de formule (4), |
| de 40 à 80 parties | d'éther butylique de triéthylèneglycol |
| de 0,5 à 5 parties | de citrate de tributyle |

15. Formulation selon l'une des revendications 11 à 14, **caractérisée en ce qu'**elle renferme, en tant que d'autres agents protecteurs contre les UV, des dérivés de l'acide p-aminobenzoique, des dérivés de l'acide salicylique, des dérivés de la benzophénone, des dérivés du dibenzoylméthane, des acrylates de diphényle, des dérivés de la benzofuranne, des dérivés de l'acide cinnamique, des dérivés camphoriques, des dérivés de la trianilino-s-triazine, des dérivés du 2-hydroxyphényl-benzotriazole ; l'acide 2-phényl-benzimidazol-5-sulfonique et leurs sels ; l'o-amino-benzoate de méthyle ; TiO₂ (différemment revêtu), ZnO et le mica.

16. Formulation selon l'une des revendications 11 à 15, **caractérisée en ce qu'**elle renferme de plus des antioxydants.

17. Utilisation de la formulation de cosmétique capillaire selon l'une des revendications 11 à 16 pour la protection de cheveux humains naturels et teints contre le rayonnement ultraviolet, **caractérisée en ce qu'**elle se présente sous forme d'un shampooing, d'une lotion, d'un gel ou d'une émulsion de rinçage à appliquer avant ou après le shampooing, avant ou après la coloration ou décoloration, avant ou après la montée d'une permanente ou un processus de défrisage, sous forme d'une lotion, d'une mousse ou d'un gel pour le frisage ou traitement, sous forme d'une lotion, d'une mousse ou d'un gel pour le brushing ou la mise en pli, sous forme d'une laque pour cheveux, sous forme d'une composition pour la montée d'une permanente, pour le défrisage, pour la coloration ou la décoloration des cheveux.

18. Procédé de traitement de cheveux humains naturels ou teints pour la protection contre l'effet de rayonnement ultraviolet, **caractérisé en ce qu'**on traite les cheveux par un shampooing, une lotion, un gel ou une émulsion de rinçage, avant ou après le shampooing, avant ou après la coloration ou la décoloration, avant ou après la montée d'une permanente ou un processus de défrisage, par une lotion, une mousse ou un gel pour le frisage, par une lotion, une mousse ou un gel pour le brushing ou la mise en pli, par une laque pour cheveux, par une composition pour la montée de permanente, pour le défrisage, pour la coloration ou la décoloration, la lotion, le gel, l'émulsion, la mousse, la laque pour cheveux ou la composition pour la permanente, pour le défrisage, pour la coloration ou la décoloration des cheveux renferme au moins un absorbeur d'UV de formule (1) ou (2).
